# EUROPEAN PATENT APPLICATION

(11) **EP 4 483 793 A1**
(43) Date of publication of application: **01.01.2025**
(21) Application number: 23181840.2
(22) Date of filing: 27.06.2023
(51) Int. Cl.: A61B 5/0215, A61B 5/00

(54) **AN INVASIVE BLOOD PRESSURE MONITORING SYSTEM**

(71) Applicant: Koninklijke Philips N.V., 5656 AG Eindhoven (NL)
(72) Inventor: WALDEN, Andreas, Eindhoven (NL); BRENKER, Sonja, Eindhoven (NL)
(74) Representative: Philips Intellectual Property & Standards

(57) **Abstract**

An invasive blood pressure monitoring system has a blood pressure transducer, a stopcock for venting a blood pressure transducer, and a display for displaying the monitored blood pressure. A controller determines if the stopcock is open or closed and provides or updates the displayed information when the stopcock is detected as open.

## Description

### FIELD OF THE INVENTION

This invention relates to invasive blood pressure monitoring systems, namely blood monitoring using a catheter inserted into the vasculature of a subject.

### BACKGROUND OF THE INVENTION

Invasive blood pressure monitoring is a commonly used method in critical care settings to measure patients' arterial blood pressure continuously. It is used in patients who require close monitoring, such as those with critical illnesses, severe injuries, or during surgical procedures.

The method involves the insertion of a catheter into an artery, which is then connected to a transducer that converts the arterial pressure into an electrical signal. This signal is then displayed on a monitor and recorded for clinical use.

The accuracy of invasive blood pressure monitoring is critical for patient safety and proper clinical management. However, inaccurate measurements can occur due to several factors, including improper system zeroing. Zeroing the invasive blood pressure system ensures that the transducer is calibrated to atmospheric pressure, which is necessary to obtain accurate measurements.

The zeroing process involves several steps, including flushing the catheter to remove any air bubbles, ensuring that the transducer is at the same level as the phlebostatic axis (the point that corresponds to the right atrium), and opening a stopcock (which vents the pressure transducer to the atmosphere) to allow the transducer to equilibrate with atmospheric pressure. The zeroing process means the recorded blood pressure traces a zero line when the stopcock is open. After the zeroing process, the clinician can verify the accuracy of the measurement by comparing it to non-invasive blood pressure readings or by performing a square wave test.

The zeroing process is a critical task that requires proper training and adherence to established protocols. It is essential to minimize the risk of complications, such as infection, bleeding, and arterial damage, resulting from improper zeroing. In addition, it is essential to ensure that the equipment is properly maintained and calibrated to ensure accurate and safe measurements.

The zeroing process includes manual tasks, which take time away from the caregiver and can lead to errors. The zeroing process requires proper training and concentration during the actual zeroing performance. In today's hospitals, caregivers are stressed because of the high workload, many tasks, and even more documentation. These circumstances can lead to delayed interventions like zeroing or errors during the procedure, leading to inaccurate invasive pressure readings. Additionally, the user interface of current patient monitors does not facilitate the zeroing procedure most efficiently.

There is a need to improve the task of zeroing an invasive blood pressure measurement.

### SUMMARY OF THE INVENTION

The invention is defined by the claims.

According to examples in accordance with an aspect of the invention, there is provided an invasive blood pressure monitoring system, comprising:
a catheter-based blood pressure transducer;
a stopcock for venting the blood pressure transducer;
a display for displaying the monitored blood pressure; and
a controller for controlling the display,
wherein the controller is configured to:
   determine if the stopcock is open or closed;
   control the display to display a trace of the sensed blood pressure and to display information relating to a zeroing function, wherein the information relating to a zeroing function is provided or updated when the stopcock is detected as open.

The zeroing function of an invasive blood pressure system ensures that the transducer is calibrated to atmospheric pressure, which is necessary to obtain accurate measurements. The need to offer a zeroing function complicates the displayed information to the user.

The system of the invention determines when the stopcock is open, in preparation for a zeroing procedure, and then adapts the display accordingly. In this way, the display does not need to be cluttered with information about the zeroing procedure before it is to be implemented.

The user interface may also include a speaker for providing audio feedback, such as voice feedback, and it may also include a microphone for receiving voice prompts to zero the invasive pressure line.

The catheter-based blood pressure transducer may have a zeroing line or it may not have a zeroing line. When there is no zeroing line, the stopcock is used to equilibrate the pressure transducer to the atmosphere. When a zeroing line is used, the stopcock is used to equilibrate the pressure transducer to the zeroing line.

Thus, the venting may be to atmospheric pressure or it may be to a zeroing line.

The controller may be configured to detect the stopcock is open based on the pressure sensed by the catheter-based blood pressure transducer. When the stopcock is manually opened, the resulting pressure changes can be sensed.

In one example, the controller is for example configured to display an icon, for actuation by a user to implement the zeroing function, adjacent or within the displayed blood pressure trace when the stopcock is detected as open; and remove the icon when the stopcock is detected as closed.

This implementation allows the user to instigate the zeroing procedure using a displayed icon. It is displayed within or next to the blood pressure trace. This makes the icon for the zeroing function most suitably placed to improve the efficiency of the zeroing procedure. The zeroing icon is removed when the system determines that the stopcock is closed.

In another example, the controller is configured to trigger an automatic zeroing procedure at after a predetermined time interval following detection of the stopcock being open.

This provides a semi-automatic procedure, which is initiated by the manual opening of the stopcock. Visual and/or audible feedback may be provided when the zeroing procedure has finished and the system can indicate if the procedure was successful or not.

In yet another example, the system comprises an actuator for actuating the stopcock, wherein the controller is configured to determine when a zeroing function is appropriate and to implement the zeroing function automatically when appropriate.

This provides an automatic procedure. The procedure for example involves recognizing when a zeroing function is needed, checking if the zeroing procedure can be performed, and then opening the stopcock automatically. This may use a servo motor for example. The system then performs the zeroing procedure and closes the stopcock automatically.

The stopcock for example comprises a sensor for sensing the stopcock position. This enables the correct operation of the actuator to be verified.

The controller may be configured to determine that the zeroing function is not appropriate based on factors including one or more of
the patient's status is critical;
there is noise above a threshold in the pressure sensed by the catheter blood pressure transducer; and
the pressure transducer is not at the correct level.

Thus, the zeroing function is allowed when it can be performed correctly and without danger to the patient.

The controller is for example configured to control the display to provide a countdown to the automatic zeroing function and provide an icon to allow cancellation of the automatic zeroing function. The countdown may be presented visually and/or acoustically.

The invention also provides a computer program comprising computer program code which is adapted, when said program is run on a controller of the blood pressure monitoring system defined above, to perform a method comprising:
receive a determination of whether the stopcock is open or closed;
controlling the display to display a trace of the sensed blood pressure and to display information relating to a zeroing function,
wherein the method comprises providing or updating the information relating to a zeroing function when the stopcock is determined to be open.

The method may further comprise:
controlling the display to display an icon, for actuation by a user to implement the zeroing function, adjacent or within the displayed blood pressure trace when the stopcock is detected as open; and
removing the icon when the stopcock is detected as closed.

The method may further comprise:
triggering an automatic zeroing procedure after a predetermined time interval following detection of the stopcock being open.

This may be considered to be a semi-automatic zeroing procedure.

The method may further comprise:
determining when a zeroing function is appropriate and implementing a zeroing function automatically when appropriate.

This may be considered to be a fully automatic zeroing procedure.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an invasive blood pressure monitoring system;
Fig. 2 shows a display output;
Fig. 3 shows one example of a display adaptation in response to the detection of the stopcock being open;
Fig. 4 shows another example of a display adaptation in response to the detection of the stopcock being open;
Fig. 5 shows a method to implement this display control functionality;
Fig. 6 shows a method to perform a timer-based semi-automatic zeroing function;
Fig. 7 shows a method to perform an automatic zeroing function; and
Fig. 8 shows an example of an actuated stopcock.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an invasive blood pressure monitoring system having a blood pressure transducer, a stopcock for venting a blood pressure transducer, and a display for displaying the monitored blood pressure. A speaker may also provide audible feedback. A controller determines if the stopcock is open or closed and provides or updates the displayed information when the stopcock is detected as open, i.e., only when the stopcock is detected as open.

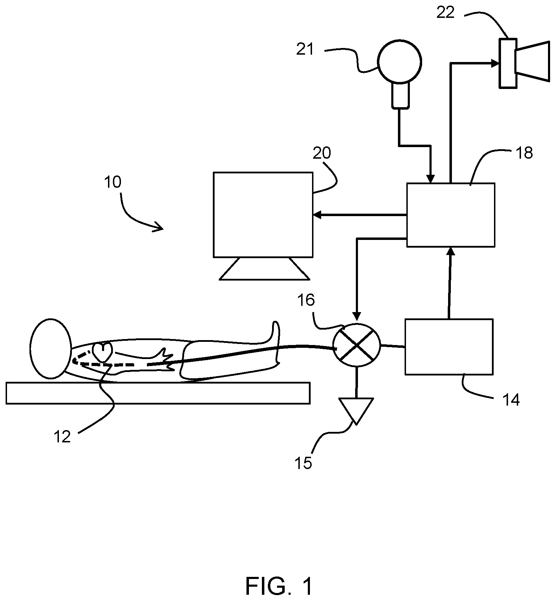

Fig. 1 shows an invasive blood pressure monitoring system 10, comprising a catheter-based blood pressure transducer having a catheter 12 and a pressure transducer 14. A stopcock 16 is provided for venting the blood pressure transducer. In the example shown, the pressure transducer 14 is vented to the ambient surroundings 15. This is suitable for a pressure transducer without a zeroing line. When a zeroing line is used, the stopcock 16 is instead used to equilibrate the pressure transducer to the zeroing line. Thus, the venting may be to atmospheric pressure or it may be to a zeroing line.

A display 20 is used to display the monitored blood pressure, under the control of a controller 18. In addition, a microphone 21 is shown for receiving user input, for example including voice commands, and a speaker 22 is shown for delivering audible output about the status of the monitored blood pressure, which may also include voice output.

The display is for example a touch screen. Other user interface elements may also be used such as a keyboard, mouse or physical buttons.

The controller also performs monitoring of the stopcock function. Thus, it determines if the stopcock 16 is open or closed.

In some examples, the stopcock is manually operated by a user, so that sensing is used to detect where the stopcock is open or closed. However, it is also possible for the controller to determine that the stopcock is open or closed because it has sent control signals to open or close the stopcock, in a fully automated implementation of the invention.

The display is used to output a trace of the sensed blood pressure and also to display information relating to a zeroing function, for example an icon which may be used to initiate the zeroing function. The information relating to a zeroing function is only provided, or is updated, when the stopcock is detected as open. This update for example means the display does not need to be cluttered with information about the zeroing procedure before it is to be implemented.

Some different implementations of the invention will now be described in more detail.

A first implementation of the invention relates to the way information about the zeroing function is configured for display (rather than the actual control of the zeroing function itself). The zeroing function thus remains a manual procedure.

An icon is typically presented to the user enabling them to instruct the system to perform a zeroing procedure. This icon is thus used when the user has placed the transducer at the correct height and opened the stopcock and hence wishes to have the pressure reading calibrated.

The zeroing function icon is dynamically placed (and removed). It is placed near the vital signs information on the screen to increase the ease of access to the user interface controls for the zeroing function and improve the efficiency of the procedure.

To enable this dynamic behavior, the analog signal received from the pressure transducer is processed by the controller to determine if the stopcock is opened (e.g., to the atmosphere). Thus, no additional sensors are needed to enable the stopcock status to be detected.

In this case, the zeroing function icon, which allows the user to zero the particular pressure line, is placed either in the corresponding vital signs area or near the area of the signal's waveform on the screen.

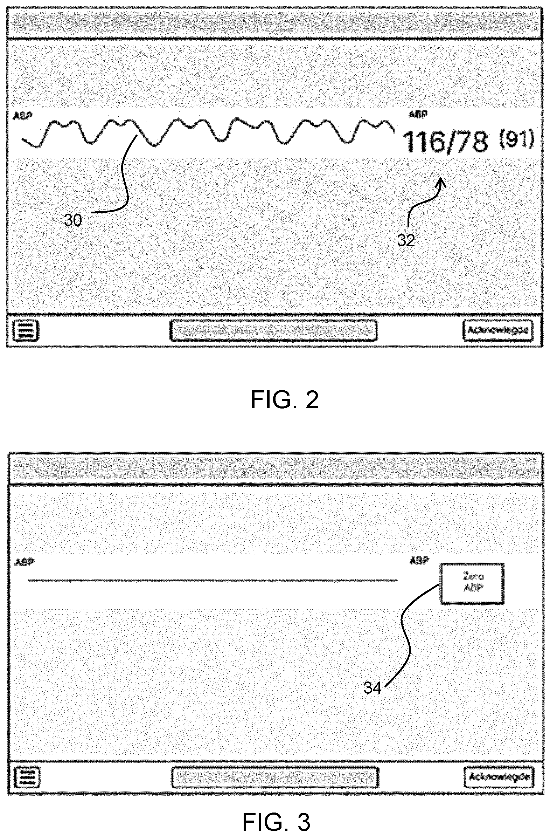

Fig. 2 shows a display output comprising the vital signs trace 30 (the arterial blood pressure, ABP) and blood pressure numeric reading 32. While the stopcock is turned off, there is no need for information on the screen about the zeroing function.

In response to the detection of the stopcock being open, one option is to place the zeroing function icon 34 next to the pressure waveform (which is now flatlining because the pressure transducer is vented). This is shown in Fig. 3.

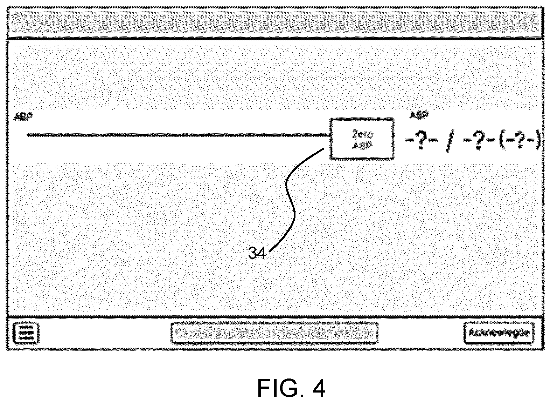

Another option is to place the zeroing function icon 34 in the flatlining pressure waveform. This is shown in Fig. 4.

The zeroing function icon is removed when the system detects that the stopcock is closed.

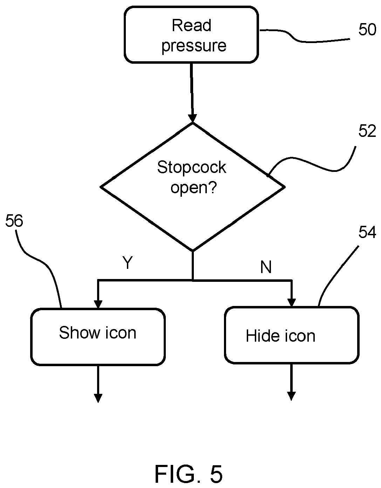

Fig. 5 shows a method to implement this functionality.

In step 50, the pressure transducer signal is read. In step 52 it is determined from the pressure reading if the stopcock is open. If not, the zeroing function icon can remain (or be made) hidden in step 54. If the stopcock is open, the zeroing function icon is displayed in step 56. The process then ends and is repeated at the next pressure transducer measurement.

The zeroing procedure may be started based on a voice command. A voice prompt, such as `zero ABP' would then only be accepted by the system while the respective stopcock is open, and the zeroing function icon is visible. This ensures safe and contextual voice command and improves efficiency as the nurse doesn't need to walk over to the patient. Sometimes the monitor is on the other side of the bed, where the arterial line and the transducer are located.

The measurement signal is processed for vital sigs interpretation with a high frequency such as with a 32ms interval. The algorithm to determine if the stopcock is open could use the same sample rate or a fraction of it. For example, a less frequent interval of 128ms would be sufficient.

A second implementation of the invention additionally relates to the way the zeroing function itself is controlled. In particular, a timer based semi-automatic zeroing function may be performed.

When it is detected that the stopcock has been opened, a timer is started, and after a countdown by the timer, the system triggers the zeroing procedure without any user interaction with the monitor. The countdown is for example in the range 1 second to 10 second, e.g., 3 seconds. The timer could be configured to the hospital needs.

The system then automatically zeros the pressure when the timer has elapsed, but the user may also still have the option to manually trigger the zeroing function. The display / speaker is controlled to visually and/or acoustically inform the user about the countdown to start the zeroing procedure. The display may also provide visual and/or audible feedback when the zeroing procedure is finished and indicates if the procedure was successful or not.

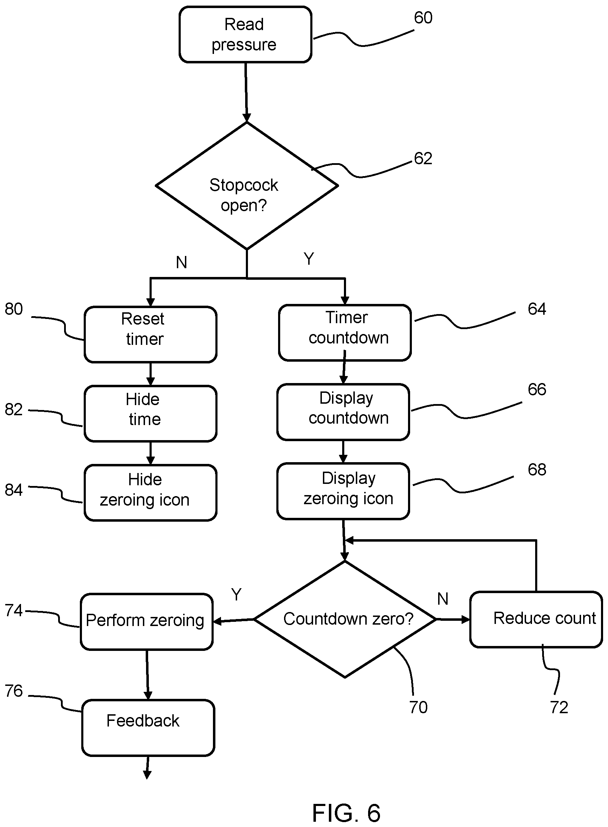

Fig. 6 shows a method to implement this functionality.

In step 60, the pressure transducer signal is read. In step 62 it is determined from the pressure reading if the stopcock is open.

If the stopcock is open, zeroing timer countdown is started in step 64. The countdown is displayed in step 66. The zeroing function icon is displayed in step 68.

In step 70 it is monitored if the countdown has reached zero. If not, the countdown is reduced a count in step 72, and this iteratively continues until countdown does reach zero.

When the countdown has reached zero, the pressuring zeroing takes place in step 74. Feedback is provided in step 76 that the zeroing function has been completed, and the process then ends, and is repeated at the next cycle of the method.

If the stopcock is detected as not open in step 62, the zeroing timer is reset in step 80. The countdown timer is hidden in step 82 and the zeroing function function icon is hidden in step 84.

A third implementation of the invention provides fully automatic zeroing functionality. In this case, the stopcock is controlled by an actuator, such as a servo motor.

A fully automated zeroing procedure uses the techniques explained above but adds the capability of automatically controlling the stopcock via an actuator and some additional logic. The actuator itself for example enables determination of the stopcock position.

Thus, by actuating the stopcock, the stopcock position is directly known without the needing to perform sensing (e.g., analysis of the pressure transducer signal) to derive the stopcock position.

For the fully automatic procedure, the system recognizes when zeroing is needed. This may be based on timing instants, or based on detecting patient position changes.

There are various situations, which require a zero:
Initial setup: When an invasive pressure line is first inserted into a patient, it needs to be zeroed before use. This is typically done after the line is connected to the pressure monitoring system and before any pressure measurements are taken.

Regular zeroing intervals: Depending on the facility's protocols, invasive pressure lines may require periodic zeroing. The frequency can vary but it is often performed at regular intervals, such as every four hours or whenever there is a significant change in the patient's position or condition. Thus, one option for automatically detecting a zero condition is simply to measure the time from the last zero.

After repositioning: If the patient's position changes, such as being moved from supine to upright or vice versa, the pressure line should be zeroed to ensure accurate measurements. This is necessary because the reference point for pressure changes with the patient's position. The patient position can also be detected automatically using camera technology, accelerometer sensors or voice documentation.

Transducer level change: If the pressure transducer is repositioned, the pressure line should be zeroed. This is important to ensure accurate measurements relative to the new reference point. Again, the transducer position could be monitored using sensors such as acceleration sensors.

When a zeroing function is needed, the system checks if the zeroing procedure can be performed (discussed below). It then opens the stopcock automatically, performs the zeroing function, and closes the stopcock automatically.

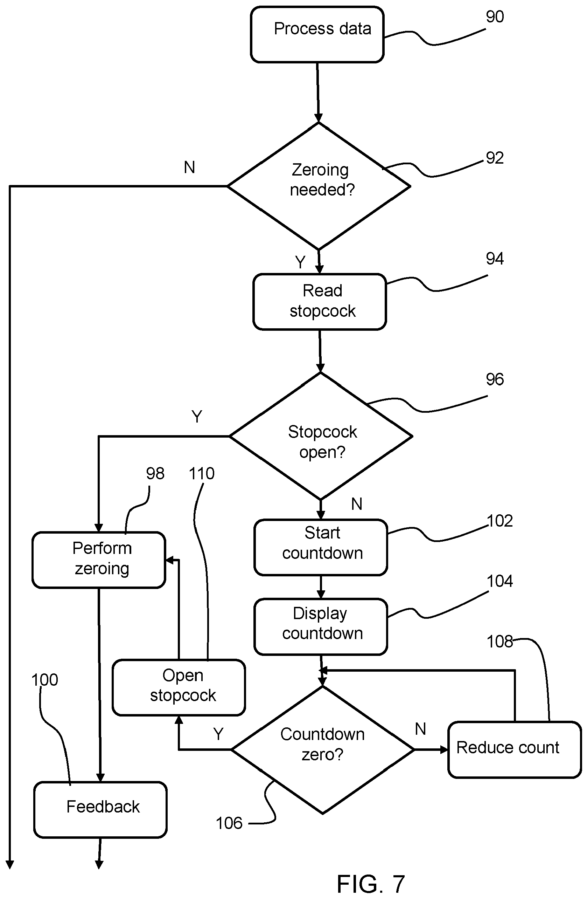

Fig. 7 shows a method to implement this functionality.

In step 90, data is processed which enables the need for zeroing to be determined, as explained above.

In step 92, it is determined if a zeroing procedure is needed and is also possible.

For safety reasons, the system will not perform auto zeroing under certain conditions. Examples include:
the patient's status is critical and physiological alarms are being derived from the monitored pressure;
the system recognizes significant noise on the transducer signal, which indicates a movement of the patient (e.g., patient transport, care procedure, etc.)
the transducer is not at the same level as the phlebostatic axis.

If zeroing is not possible or not needed, the process ends, until the next repeat iteration of the process.

If zeroing is needed and possible, the stopcock position is read in step 94 based on information from the stopcock actuator.

It is then determined in step 96 if the stopcock is open. If it is open, the zeroing function takes place in step 98. Feedback is then provided in step 100 via the display / speaker that the zeroing function has been performed and was successful. The process then ends, until the next repeat iteration of the process.

It is known how to determine if zeroing has been successful, by using plausibility checks, such as a stable signal during zero procedure (since the atmosphere pressure is relatively stable). The value of the calibrated pressure reading relative to the atmospheric pressure should be within a certain range.

If the stopcock is not detected as open in step 96, a zeroing timer countdown is started in step 102. The countdown is displayed in step 104.

The countdown offers the user the chance to cancel the procedure manually.

In step 106 it is monitored if the countdown has reached zero. If not, the countdown is reduced a count in step 108, and this iteratively continues until countdown does reach zero.

When the countdown has reached zero, the stopcock is opened in step 110. The zeroing function then takes place in step 98. Feedback is again provided in step 100 via the display / speaker that the zeroing function has been performed and was successful. The process then ends, until the next repeat iteration of the process.

In the semi-automated or fully-automated approaches described above, the stopcock can preferably still be operated manually. A manual closing of the stopcock for example may cancel and thereby prevent the system from performing a zeroing procedure.

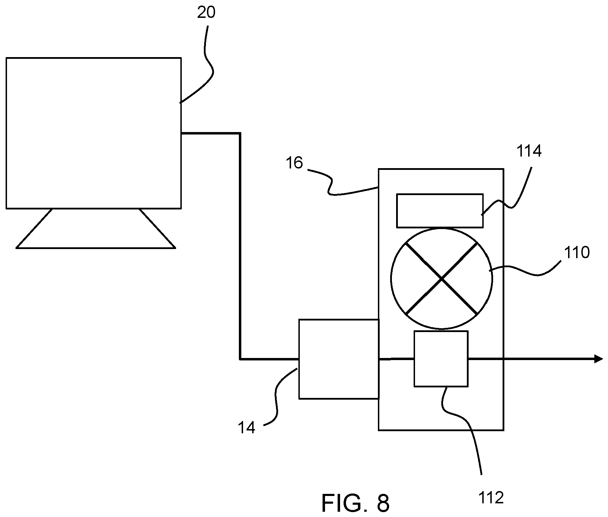

An example of the actuated stopcock is shown in Fig. 8. The stopcock 16 comprises a valve 110, a servo motor 112 for driving the valve and a position sensor 114 for sensing the valve position.

The system sends commands to the stopcock, which controls the servo motor 112 to open or close the stopcock and enables the zeroing procedure. Furthermore, the sensor 114, which provides data on the position of the stopcock. The system can receive this data to detect if the position is in the desired position. Thus, it verifies the correction operation of the actuator.

Thus, the sensor is not needed to determine the position of the stopcock, since it should be known from the actuator drive signals. The sensor is used for checking correct operation of the stopcock.

The system saves the current zeroing information before starting a new auto zeroing function, so that the system can recover from a failed zeroing attempt, and inform the caregiver through a notification.

As mentioned above, the system needs to be flushed while setting up the catheter. When an actuated and sensed valve is used, a flushing function could also be automated, to automatically flush, detect air bubbles vs. fluid and close automatically.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An invasive blood pressure monitoring system (10), comprising:
a catheter-based blood pressure transducer (12,14);
a stopcock (16) for venting the blood pressure transducer;
a display (20) for displaying the monitored blood pressure; and
a controller (18) for controlling the display,
wherein the controller (18) is configured to:
determine if the stopcock is open or closed;
control the display to display a trace (30) of the sensed blood pressure and to display information (34) relating to a zeroing function, wherein the information relating to a zeroing function is provided or updated when the stopcock is detected as open.

2. The system of claim 1, wherein the stopcock (16) is for venting the blood pressure transducer (14) to the ambient surroundings.

3. The system of claim 1, wherein the blood pressure transducer (14) comprises a zeroing line, and the stopcock (16) is for venting the blood pressure transducer to the zeroing line.

4. The system of any one of claims 1 to 3, wherein the controller is configured to detect the stopcock is open based on the pressure sensed by the blood pressure transducer.

5. The system of any one of claims 1 to 4, wherein the controller is configured to:
display an icon (34), for actuation by a user to implement the zeroing function, adjacent or within the displayed blood pressure trace when the stopcock is detected as open; and
remove the icon (34) when the stopcock is detected as closed.

6. The system of any one of claims 1 to 4, wherein the controller is configured to:
trigger an automatic zeroing procedure after a predetermined time interval following detection of the stopcock being open.

7. The system of any one of claims 1 to 4, further comprising an actuator (112) for actuating the stopcock, wherein the controller is configured to determine when a zeroing function is appropriate and to implement the zeroing function automatically when appropriate.

8. The system of claim 7, wherein the stopcock comprises a sensor (114) for sensing the stopcock position.

9. The system of claim 7 or 8, wherein the controller is configured to determine that the zeroing function is not appropriate based on factors including one or more of:
the patient's status is critical;
there is noise above a threshold in the pressure sensed by the catheter blood pressure transducer; and
the pressure transducer is not at the correct level.

10. The system of claim 7, 8 or 9, wherein the controller is configured to control the display to provide a countdown to the automatic zeroing function and provide an icon to allow cancellation of the automatic zeroing function.

11. A computer program comprising computer program code which is adapted, when said program is run on a controller of the blood pressure monitoring system of any one of claims 1 to 10, to perform a method comprising:
receive a determination of whether the stopcock is open or closed;
controlling the display to display a trace (30) of the sensed blood pressure and to display information (34) relating to a zeroing function,
wherein the method comprises providing or updating the information relating to a zeroing function when the stopcock is determined to be open.

12. The computer program of claim 11, wherein the method further comprises:
controlling the display to display an icon (34), for actuation by a user to implement the zeroing function, adjacent or within the displayed blood pressure trace when the stopcock is detected as open; and
removing the icon (34) when the stopcock is detected as closed.

13. The computer program of claim 11, wherein the method further comprises:
triggering an automatic zeroing procedure after a predetermined time interval following detection of the stopcock being open.

14. The computer program of claim 11, wherein the method further comprises:
determining when a zeroing function is appropriate and implementing a zeroing function automatically when appropriate.
